# EUROPÄISCHE PATENTSCHRIFT

(11) **EP 1 244 491 B1**
(45) Veröffentlichungstag und Bekanntmachung des Hinweises auf die Patenterteilung: **21.09.2005**
(21) Anmeldenummer: 00993239.3
(22) Anmeldetag: 01.12.2000
(51) Int. Cl.: A61M 25/01, A61M 25/06, A61B 8/08, A61M 5/32

(54) **VORRICHTUNG ZUM EINFÜHREN DES DISTALEN ENDES EINER KANÜLE IN EIN GEFÄSS**
DEVICE FOR INSERTING THE DISTAL END OF A HOLLOW NEEDLE IN A BLOOD VESSEL
DISPOSITIF POUR INTRODUIRE L'EXTREMITE DISTALE D'UNE CANULE DANS UN VAISSEAU

(30) Priorität: 06.12.1999 DE 19958688
(43) Veröffentlichungstag der Anmeldung: 02.10.2002
(73) Patentinhaber: Heidrich, Jens Peter, 23558 Lübeck (DE)
(72) Erfinder: Heidrich, Jens Peter, 23558 Lübeck (DE)
(74) Vertreter: Vollmann, Heiko
(86) Internationale Anmeldenummer: PCT/DE2000/004310
(87) Internationale Veröffentlichungsnummer: WO 2001/039824

(56) Entgegenhaltungen:
- CH-A- 676 787
- DE-A- 4 206 065
- DE-B- 1 055 760
- DE-B- 1 300 198
- DE-U- 9 414 727
- US-A- 5 207 647

## Beschreibung

Die Erfindung betrifft eine Vorrichtung zum Einführen einer elastisch biegsamen Verweilkanüle zusammen mit einer darin befindlichen im Wesentlichen starren Punktionskanüle in ein Blutgefäß eines menschlichen oder tierischen Körpers.

Das Einführen von Kanülen in Blutgefäße zählt zu den Grundfertigkeiten eines jeden Arztes. Obwohl dies täglich zigtausendfach durchgeführt wird, treten oftmals Probleme dabei auf, und zwar sowohl beim Einstechen der im Wesentlichen als starr zu betrachtenden Kanülen, wie sie beispielsweise bei Spritzen vorgesehen sind, als auch insbesondere bei den größerlumigen Verweilkanülen, die aus einem starren Teil zum Punktieren und einem biegeelastischen Teil zum anschließenden Verbleib im Gefäß bestehen. Die bisher üblichen manuellen Verfahren sind in hohem Maße von der subjektiven Beurteilung und vom Geschick des behandelnden Arztes abhängig, das Blutgefäß zu finden und zu treffen. Häufig bereiten auch besondere anatomische Verhältnisse, Adipositas oder krankhafte Zustände wie beispielsweise Ödeme, Schwierigkeiten. Aufgrund der gegebenen Größenverhältnisse und Gewebeeigenschaften ist das Einführen von Verweilkanülen bei Säuglingen und Kleinkindern besonders schwierig. Der Verlauf der Venen lässt sich häufig nur erahnen. Zwar sind schon einige Vorrichtungen vorgeschlagen worden, die das Auffinden und Einführen einer Spritzenkanüle erleichtern sollen, sie haben sich jedoch in der Praxis nicht durchsetzen können und sind für das Einsetzen von Verweilkanülen wenig geeignet.

Aus US-PS 5,311,871 ist eine Vorrichtung bekannt, bei der im Kolbenboden einer Spritze ein Ultraschallerzeuger angeordnet ist. Die dort erzeugten Schwingungen werden über das im Spritzenzylinder befindliche Fluid auf die Kanüle übertragen, wobei der Sender gleichzeitig Empfänger für die reflektierten Schallwellen ist und die Dopplersonographie eingesetzt wird, um ein Blutgefäß aufzufinden. Eine ähnliche Anordnung ist auch aus US-PS 5,080,103 bekannt. Diese Ortungshilfen haben sich in der Praxis jedoch nicht durchsetzen können, da das Ortungsprinzip erst funktioniert, wenn die Kanüle innigen Kontakt mit dem Körper hat, d. h. bereits die Körperoberfläche durchdrungen hat. Für dicht unter der Oberfläche der Haut verlaufende Gefäße sind sie demnach ungeeignet.

Aus US-PS 5,427,108 ist eine Vorrichtung bekannt, mit der ein Blutgefäß ebenfalls unter Anwendung der Ultraschall-Dopplersonographie geortet werden kann, wobei die Vorrichtung dabei so in Richtung zum Gefäß ausgerichtet wird, dass die Kanüle durch eine Führung der Vorrichtung manuell in das Gefäß eingeführt werden kann. Noch weitergehend mechanisiert ist eine aus US-PS 4,527,569 bekannte Vorrichtung, bei der eine Halterung für die einzuführende Kanüle weitgehend selbsttätig in Bezug auf das geortete Blutgefäß positioniert wird und das Einführen der Kanüle in das Gefäß dann manuell gesteuert erfolgt. Die beiden letztgenannten Vorrichtungen sind technisch aufwendig und haben sich in der Praxis ebenfalls nicht bewährt. Zwar kann die Ortung von Gefäßen mit solchen Vorrichtungen erleichtert werden, das Einführen der Kanüle selbst jedoch wird kaum vereinfacht, da der Arzt den Gefäßverlauf abschätzen und daher die Einstechtiefe selbst bestimmen muss.

Aus DE 41 42 795 C1 ist eine Vorrichtung zum Injizieren oder Punktieren von Gelenkhohlräumen bekannt, bei der eine Punktionsspritze in einer Spritzenhalterung gelagert ist, die an einem Träger elektromotorisch verfahrbar ist. Die Motorsteuerung erfolgt in Abhängigkeit des Signals einer Sonarmessvorrichtung derart, dass stets die richtige Einstichtiefe der an der Spritze angeordneten Kanüle gewährleistet ist. Ungeachtet dessen, dass diese Vorrichtung zum Punktieren von Gelenkhohlräumen vorgesehen ist, ist sie weder zum Einführen von Kanülen und noch viel weniger zum Einführen von Verweilkanülen in Gefäße geeignet, da sie im Wesentlichen senkrecht zur Körperoberfläche die Nadel einführt und lediglich die Einstichtiefe erfasst. Darüber hinaus ist der die Kanüle umgebende Ultraschallmesskopf für die Gefößpunktion wenig geeignet, da er die Einstichstelle auf der Haut völlig verdeckt und somit eine Sichtkontrolle verhindert, die zweckmäßigerweise stets erfolgen können sollte.

Aus DE 42 06 065 C2 ist eine Hilfsvorrichtung zum Einführen einer Kanüle einer Spritze bekannt, bei der zwei Schallköpfe vorgesehen sind, so dass die Lage und der Verlauf des zu punktierenden Gefäßes recht zuverlässig erfasst werden können. Es handelt sich hierbei jedoch um eine reine Ortungshilfe, eine Mechanisierung des Punktionsvorgangs ist bei dieser Vorrichtung nicht vorgesehen, so dass der eigentliche Punktionsvorgang nicht reproduzierbar ist und nach wie vor dem manuellen Geschick des Arztes obliegt.

Die aus DE 94 14 727 U1 bekannte Vorrichtung dient zur Biopsie, also zur Entnahme von Gewebeproben und ist daherfür das hier in Rede stehende Anwendungsgebiet nicht einsetzbar.

All den vorerwähnten bekannten Vorrichtungen gemeinsam ist, dass sie entweder nur Hilfsvorrichtungen beim Einführen einer Kanüle in ein Gefäß darstellen und daher keine reproduzierbaren Ergebnisse liefern oder aber für das mechanisierte Einführen einfacher Kanülen vorgesehen sind, sich also nicht zum Einführen von Verweilkanülen eignen, bei denen zwei Kanülen einzuführen und dann in bestimmungsgemäßer Art relativ zueinander zu bewegen sind.

Vor diesem Stand der Technik liegt der Erfindung die Aufgabe zugrunde, eine Vorrichtung zum Einführen einer elastisch biegsamen Verweilkanüle mit einer im Wesentlichen starren Punktionskanüle zu schaffen, die das Legen einer Verweilkanüle weitgehend mechanisiert und automatisiert.

Diese Aufgabe wird gemäß der Erfindung durch die in Anspruch 1 angegebenen Merkmale gelöst. Vorteilhafte Ausgestaltung der Erfindung sind in den Unteransprüchen der nachfolgenden Beschreibung und der Zeichnung angegeben.

Um die Zuverlässigkeit und Reproduzierbarkeit des Einführvorgangs möglichst unabhängig von der manuellen Geschicklichkeit und den gegebenenfalls vorhandenen anatomischen Besonderheiten zu machen, sieht die Erfindung vor, dass nicht nur eine Ortungseinrichtung als Hilfe zum Auffinden des Gefäßes und eine Halterung für die einzuführende Kanüle vorgesehen werden, sondern dass der gesamte Einführvorgang mechanisiert wird, indem für die Verweilkanüle und für die Punktionskanüle jeweils eine gesonderte Halterung vorgesehen sind, die längs einer Führung unabhängig voneinander in Einführrichtung sowie Gegenrichtung verfahrbar sind. Die Steuerung erfolgt in Abhängigkeit des Ausgangssignals der Ortungseinrichtung, die sowohl zum Auffinden des Gefäßes als auch zur Erfassung des Gefaßverlaufes, und zwar vor und hinter der Punktionsstelle bzw. der zu erwartenden Punktionsstelle der Punktionskanüle ausgebildet ist. Da die Halterungen unabhängig voneinander verfahrbar sind, können nicht nur die Verweilkanüle mit der darin befindlichen Punktionskonüle in das Gefäß eingeführt werden, sondern darüber hinaus auch die Puntkionskanüle zurückgezogen und dabei die Verweilkanüle gegebenenfalls weiter vorgeschoben werden, wie dies auch beim manuellen Legen solcher Verweilkanülen üblich ist.

Grundsätzlich können mit der erfindungsgemäßen Vorrichtung auch einfache Kanülen in ein Gefäß sicher und reproduzierbar eingeführt werden, doch besonders vorteilhaft ist die Anwendung für das Legen von Verweilkanülen. Verweilkanülen weisen üblicherweise ein im Vergleich zu Spritzenkanülen wesentlich größeres Lumen auf, so dass es für diese Kanülen noch wichtiger ist, präzise und schnell in die bestimmungsgemäße Lage gebracht zu werden, da Schmerzen, Verletzungsgefahr und deren Folgen beim Einführen von Verweitkanülen deutlich größer sind als bei anderen Kanülen. Neben der vollständigen Mechanisierung des Einführvorgangs bietet die erfindungsgemäße Vorrichtung weiterhin den Vorteil, dass aufgrund der steuerungstechnischen Verknüpfung zwischen dem Ausgangssignal der Ortungseinrichtung und den Antriebsmitteln zum Einführen der Kanülen auch tiefer gelegene Blutgefäße sicher erreicht werden können. Dabei kann durch entsprechende Abstimmung der Einführgeschwindigkeit sowie dadurch, dass stets in einem günstigen Winkel eingeführt wird, das Schmerzempfinden des Patienten herabgesetzt werden. Des weiteren kann durch das sichere und punktgenaue Einführen die Gefahr von Gefäßwandverletzungen und deren Folgeerscheinungen wie Blutungen, Hämatome oder Gefäßentzündungen deutlich vermindert werden. Schließlich wird durch die Mechanisierung des Einführvorgangs auch das Infektionsrisiko für die behandelnde Person herabgesetzt, da eine manuelle Führung derscharfen Kanüle während der Punktion vermieden wird und der Abstand zum Patienten vergrößert wird, so dass die behandelnde Person weniger leicht mit spritzendem Blut in Kontakt kommt.

Bevorzugt weist die Vorrichtung zwei Ortungseinrichtungen auf, die so ausgerichtet sind, dass das zu punktierende Gefäß derart geortet wird, dass die Punktionsstelle und ein Bereich in Enstichrichtung hinter der Punktionsstelle detektiert wird, damit der gesamte Einführvorgang gemäß des Gefäßverlaufes zuverlässig unter Ortungskontrolle erfolgen kann. Die Ortungseinrichtungen erfassen aber auch den Bereich vor der Punktionsstelle, so dass der Gefäßverlauf in unmittelbarer Umgebung der Punktionsstelle bzw. die zu erwartende Punktionsstelle detektiert und auch während des Einführvorgangs überwacht wird.

Als Ortungseinrichtung dient ein Ultraschallsender und -empfänger, der vorzugsweise dopplersonographisch arbeitet, da dann aufgrund der Strömung innerhalb der Gefäße diese von anderen Organen besonders gut unterscheidbar sind und darüber hinaus anhand der Flussrichtung auch ohne weiteres festgestellt werden kann, ob es sich bei dem gerade georteten Gefäß um eine Vene oder um eine Arterie handelt. Derartige Ultraschallortungssysteme sind hinlänglich bekannt, arbeiten entweder kontinuierlich (CW-Dopplersonographie) oder aber gepulst (PW-Dopplersonographie). Sie sind auf dem Markt verfügbar und werden daher hier nicht im Einzelnen beschrieben. Es wird in diesem Zusammenhang auf Schäberle, Wilhelm, Ultraschall in der Gefäßdiagnostik, Springer Verlag, Berlin, Heidelberg 1998 (ISBN 3-540-63148-8) verwiesen.

Um sicherzustellen, dass das Einführen der Kanüle nur dann erfolgt, wenn die Vorrichtung sich in der gegenüber dem zu punktierenden Gefäß bestimmungsgemäßen Lage befindet, ist die Steuerung so ausgelegt, dass der die Kanülen in Einführrichtung bewegende Antrieb nur dann freigeschaltet ist, wenn die Auswertung der von den Ortungseinrichtungen abgegebenen Signale durch die Steuerung ergibt, dass sich die Vorrichtung in einer für den Punktionsvorgang geeigneten Stellung zu dem zu punktierenden Gefäß befindet. Um dem Therapeuten eine Hilfestellung bei der Ausrichtung der Vorrichtung zum Patienten zu geben, ist eine optische oder akustische Anzeige vorgesehen, welche ein Signal abgibt, wenn sich die Vorrichtung in der bestimmungsgemäßen Lage zur Punktion des georteten Gefäßes befindet. Eine solche Anzeige kann beispielsweise durch Leuchtdioden gebildet sein, die programmgesteuert durch optisches Signal angeben, wenn die optimale Lage der Vorrichtung zum Gefäß zum Einbringen der Kanüle erreicht ist. Dazu wird die Vorrichtung manuell über die Haut bewegt, bis die Anzeige aufleuchtet.

Um eine Groborientierung der Vorrichtung beim Anlegen zu erleichtern, ist es zweckmäßig, das distale Ende der Vorrichtung mit einer Anlagefläche auszustatten, die in Richtung auf die Verfahrrichtung der Führungen, also der Bewegungsrichtung der Kanülenhalterungen, schräg gestellt ist. Der Winkel dieser Schrägstellung liegt zweckmäßigerweise zwischen 30° und 45°, also in dem Bereich, in dem eine optimale Ausrichtung der Kanülen in Bezug auf das zu punktierende Gefäß zu erwarten ist, wenn diese Anlagefläche zur Anlage auf der Haut eines Patienten, beispielsweise im Bereich der Extremitäten, gebracht wird. Es versteht sich, dass dieser Winkel in Hinblick auf die Form des distalen Kanülenendes angepasst sein kann. Um jedoch trotz einer flächigen Anlage dem Anwender die Möglichkeit zu geben, die Vorrichtung in Bezug auf das geortete Gefäß auszurichten, ist es zweckmäßig, die Anlagefläche nicht völlig flach, sondern ballig auszubilden, so dass zumindest in den zu erwartenden Grenzen die Vorrichtung nach vorne, nach hinten oder auch zu der einen oder anderen Seite geschwenkt werden kann. Auch erleichtert die ballige Ausgestaltung das Gleiten über die Haut zum Auffinden des Gefäßes. Insbesondere beim Einsatz von Ultraschallortungseinrichtungen ist es erforderlich, dass zwischen Sender und dem zu ortenden Objekt bzw. zwischen Empfänger und dem zu ortenden Objekt eine gute Ankopplung, d. h. eine gut Schallwellen leitende Verbindung besteht. Um dies zu erreichen, ist es zweckmäßig, die Senderempfängeranordnungen rückseitig der Anlagefläche anzuordnen.

Es ist zweckmäßig, zwei Linearführungen vorzusehen, nämlich eine für die Punktionskanüle und eine weitere parallel dazu angeordnete für die Verweilkanüle. Die Antriebe sind dabei getrennt ansteuerbar, um auch den Vorgang des Zurückziehens der Punktionskanüle sowie des Vorschiebens der Verweilkanüle weitgehend mechanisiert durchführen zu können.

Handelsübliche Verweilkanülen besitzen zur Punktion in der Regel eine starre, scharf geschliffene Punktionskanüle, die von der eigentlichen stumpfen, elastisch biegsamen Verweilkanüle umgeben ist, die gemeinsam mit der Punktionskanüle in das Gefäß eingeführt wird. Nach der Punktion wird die Verweilkanüle in Einführrichtung weiter vorgeschoben und die Punktionskanüle zurückgezogen und schließlich manuell entfernt.

Je nach Einsatzzweck kann die Vorrichtung unterschiedliche Formen aufweisen. Als besonders vorteilhaft hat sich eine etwa pistolenförmige Ausbildung der Vorrichtung erwiesen, bei der eine Auslösetaste im vorderen Teil des Griffstückes vorgesehen und die Steuer- und Auswertelektronik vorzugsweise ebenfalls im Griffstück integriert ist. Die zum Betrieb der Vorrichtung erforderliche elektrische Energie wird zweckmäßigerweise durch einen im Griffstück oder an anderer geeigneter Stelle angeordneten Akku bereitgestellt, wobei wie bei derartigen Geräten üblich eine Ladehalterung oder auch eine externe Ladestation für den Akku vorgesehen sein kann. Eine solche Anordnung ist insbesondere dann zweckmäßig, wenn die Vorrichtung einhändig gehalten und geführt werden soll. Die pistolenartige Ausgestaltung wird gewählt, um möglichst flexibel jedes erreichbare Gefäß an Rumpf oder Extremitäten punktieren zu können. Die Betätigung mit nur einer Hand gibt dem Anwender jederzeit die Möglichkeit, mit der anderen Hand die Extremität des Patienten zu halten und ggf. schnell korrigierend oder unterstützend einzugreifen. Nach dem Einsetzen der Verweilkanüle wird die freie Hand benötigt, um die Halterungen von der Verweilkanüle zu lösen und die Punktionskanüle zu entnehmen und entsprechende Leitungsverbindungen für zum Beispiel eine Infusion herzustellen.

Die an den Führungen vorgesehenen Halterungen für die Punktionskanüle und für die Verweilkanüle müssen mit Antriebsmitteln versehen sein. Dabei wird es als besonders vorteilhaft angesehen, die Führungen außenliegend am Gehäuse vorzusehen und den eigentlichen Antrieb nach innen in den Pistolengriff zu verlegen und lediglich jeweils einen Mitnehmer über eine Längsausnehmung in den pistolenartigen Körperzum Zwecke der Antriebsverbindung eingreifen zu lassen. Dies ist schon aus Gründen der Hygiene zweckmäßig, da sämtliche Bauteile, die mit den Kanülen in Verbindung treten können, zum Zwecke der Reinigung und Desinfektion zugänglich und abnehmbar sein sollten. Der Antrieb erfolgt dabei vorzugsweise mittels Elektromotoren, die beispielsweise eine Schnecke antreiben, in die ein mit der Halterung verbundener Mitnehmer eingreift, so dass die Halterung längs der Führung durch Wahl der Drehrichtung und Anzahl der Drehungen des Motors definiert verfahrbar ist. Anstelle eines Schneckenantriebs kann auch ein Teleskopantrieb Verwendung finden. Derartige Antriebe sind beispielsweise zum Ein- und Ausfahren von Teleskopen von Autoantennen bekannt.

Um sicherzustellen, dass die Kanülen entsprechend ihrer Bauart und Größe bestimmungsgemäß eingeführt werden, ist es zweckmäßig an der Vorrichtung entsprechende Sensorik zur Erfassung von Kanülentyp und/oder-größe vorzusehen. Da die Kanülen üblicherweise mit größer werdenden Lumen auch in ihrer Baulänge wachsen kann die jeweils eingesetzte Kanülengröße beispielsweise durch eine auf der Oberseite angebrachte Reihe von Fotodioden, die nach Art einer Reflektionslichtschranke arbeiten, ermittelt und der Steuerung mitgeteilt werden. Alternativ kann auch ein Scanner vorgesehen sein, der einen kanülenseitig aufgebrachten Barcode erfasst, in dem der Kanülentyp und die Kanülengröße verschlüsselt angegeben sind. Auch eine mechanische Erfassung ist denkbar, sei es, dass eine Vorrichtung nur zum Einsatz eines bestimmten Kanülentyps ausgebildet ist oder dass die Größenunterschiede mechanisch abgetastet werden.

Die Erfindung ist nachfolgend anhand eines in der Zeichnung dargestellten Ausführungsbeispiels erläutert. Es zeigen:
- Fig. 1: in schematischer Darstellung eine Seitenansicht einer erfindungsgemäßen Vorrichtung,
- Fig. 2: eine Draufsicht auf die Vorrichtung nach Fig. 1,
- Fig. 3: eine Ansicht einer gebräuchlichen Verweilkanüle mit Punktionskanüle,
- Fig. 4: eine um 90° in Bezug auf die Kanülenachse versetzte Ansicht der Punktionskanüle nach Fig. 3,
- Fig. 5: die Verweilkanüle in Darstellung nach Fig. 4,
- Fig. 6: die Anordnung des distalen Vorrichtungsendes mit den Ortungseinrichtungen in Bezug auf ein Gefäß, das zu punktieren ist,

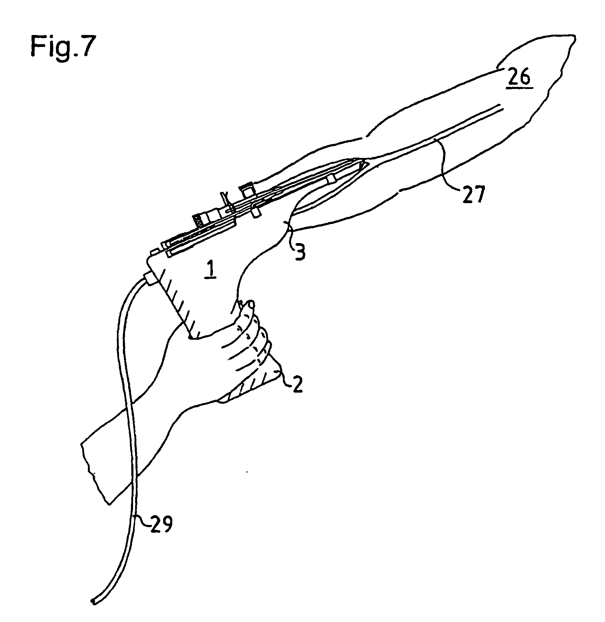

- Fig. 7: eine schematische perspektivische Darstellung der Vorrichtung beim Einsatz an einem Arm,
- Fig. 8: die Anordnung des distalen Vorrichtungsendes zu Beginn des Einführungsvorgangs und
- Fig. 9: die Anordnung gemäß Fig. 8 während des Vorschiebens der Verweilkanüle über die Punktionskanüle hinweg in das Gefäß.

Die in den Figuren dargestellte Einführvorrichtung weist ein pistolenförmiges Gehäuse 1 auf, mit einem Griffteil 2 zur Handhabung der Vorrichtung. Das Gehäuse 1 geht nach oben in einen laufförmigen Teil 3 über, dessen distales Ende 4 eine Anlagefläche 5 aufweist.

Auf der Oberseite des laufähnlichen Teils 3 ist eine erste teleskopartige Führung 6 vorgesehen, die zwei, mit Abstand und parallel zueinander angeordnete Stangen 7 aufnimmt, welche mittels eines nicht im Einzelnen dargestellten elektromotorischen Antriebs in Achsrichtung der Stangen 7 verfahrbar sind. Am distalen Ende der Stangen 7 ist eine Halterung 8 zur lösbaren Aufnahme einer Punktionskanüle 9 vorgesehen.

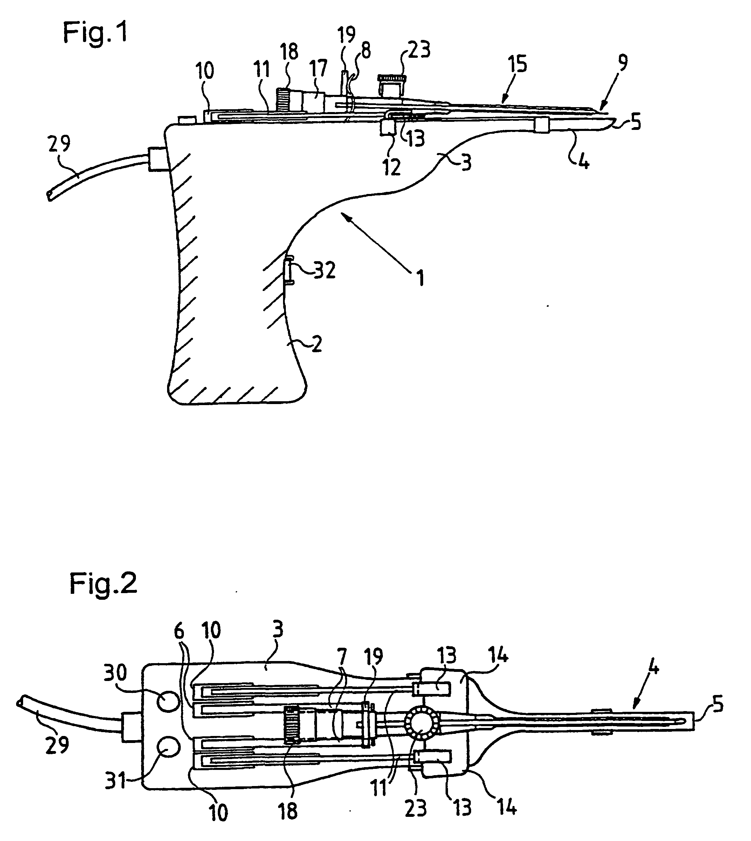

Parallel zu der Führung 6 ist an der Oberseite des laufähnlichen Teils 3 eine weitere Führung 10 vorgesehen, deren ebenfalls teleskopierbare Stangen 11 parallel zueinander und parallel zu den Stangen 7 angeordnet sind. Die Stangen 11 weisen, wie in Fig. 1 angedeutet, Mitnehmer 12 auf, die durch Längsausnehmungen mit einem ebenfalls innerhalb des Griffteils 2 angeordneten elektromotorischen Antrieb verbunden sind. An den distalen Enden der Stangen 11 sind klippartige Halterungen 13 vorgesehen, mit denen Flügel 14 einer Verweilkanüle 15 gehalten werden.

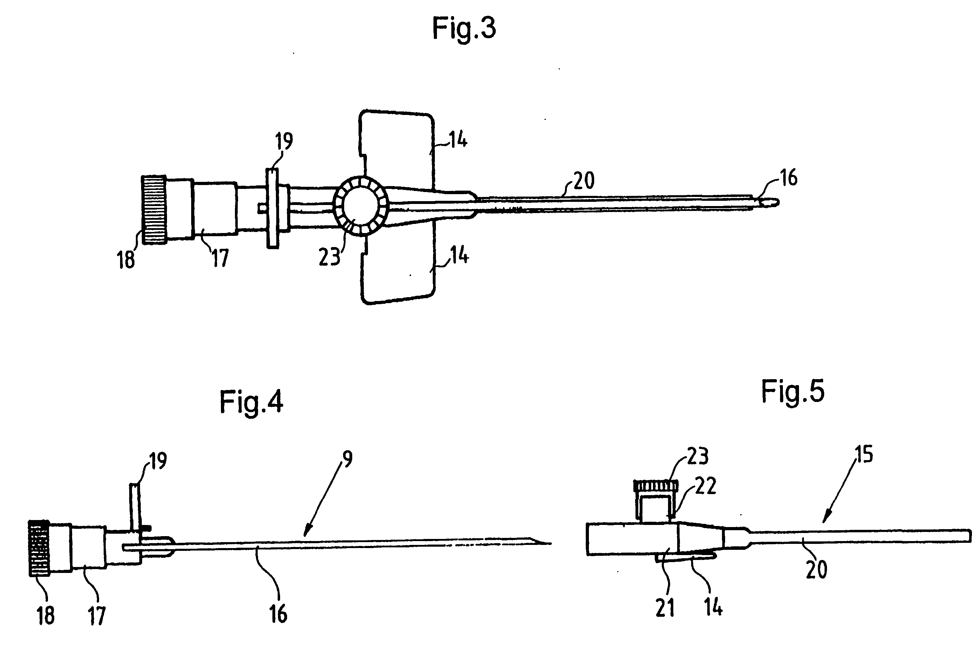

Bei der in den Figuren dargestellten Kanülen 9, 15 handelt es sich um übliche Verweilkanülen, wie sie beispielsweise unter der Marke VENFLON der Firma VIGGO AG, Schweden, im Handel und anhand der Figuren 3 bis 5 im Einzelnen dargestellt sind. Eine solche Kanülenanordnung besteht aus einer im Wesentlichen starren Punktionskanüle 9, wie sie in Fig. 4 dargestellt ist, und aus einer elastisch biegbaren Kunststoffverweilkanüle 15, wie sie in Fig. 5 dargestellt ist. Die Punktionskanüle besteht aus einem üblichen distalseitig schräg angeschliffenen Metallrohr 16, dessen proximales Ende in einem Kunststoffteil 17 gehalten ist, das proximalseitig als Lueranschluss ausgebildet und mittels eines Verschlussstopfens 18 abgeschlossen ist. An dem Kunststoffteil 17 ragt ein Führungsteil 19 nach oben heraus, es dient der manuellen Entfernung der Punktionskanüle aus der Verweilkanüle nach erfolgreicher Punktion des Gefäßes.

Die biegeelastische Verweilkanüle 15 besteht aus einem flexiblen Kunststoffrohr 20, das proximalseitig ebenfalls in einem Kunststoffteil 21 mündet, dessen proximales Ende auch als Lueranschluss ausgebildet ist. Darüber hinaus weist das Kunststoffteil 21 noch einen Querzugang 22 auf, der mittels eines Verschlussstopfens 23 abschließbar ist. Die Kanülen 9 und 15 sind so dimensioniert, dass sie in zusammengesetzter Form die in Fig. 3 dargestellte Anordnung bilden, bei der das distale, angeschliffene freie Ende des Metallrohrs 16 das distale freie Ende des Kunststoffrohrs 20 überragt.

Die Kanülen 9 und 15 werden in der in Fig. 3 dargestellten Anordnung in der Vorrichtung klemmbefestigt, so wie dies anhand der Figuren 1 und 2 dargestellt ist. Dabei werden die Führung 19 der Kanüle 9 in der Halterung 8 und die Flügel 14 in den Halterungen 13 befestigt. Die Stangen 7 und 11 sind, wenn sie in Ausgangssituation vollständig eingefahren sind, so wie es in den Figuren 1 und 2 dargestellt ist, so ausgelegt, dass das distale Ende des Metallrohrs 16 vor der Anlagefläche 5, also vor dem distalen Ende der Vorrichtung endet. Die Kanülen 9 und 15 sind dabei nur in den Halterungen 8 und 13 befestigt, im Übrigen jedoch mit Abstand zum Gehäuse 1 angeordnet, um Sterilität zu sichern.

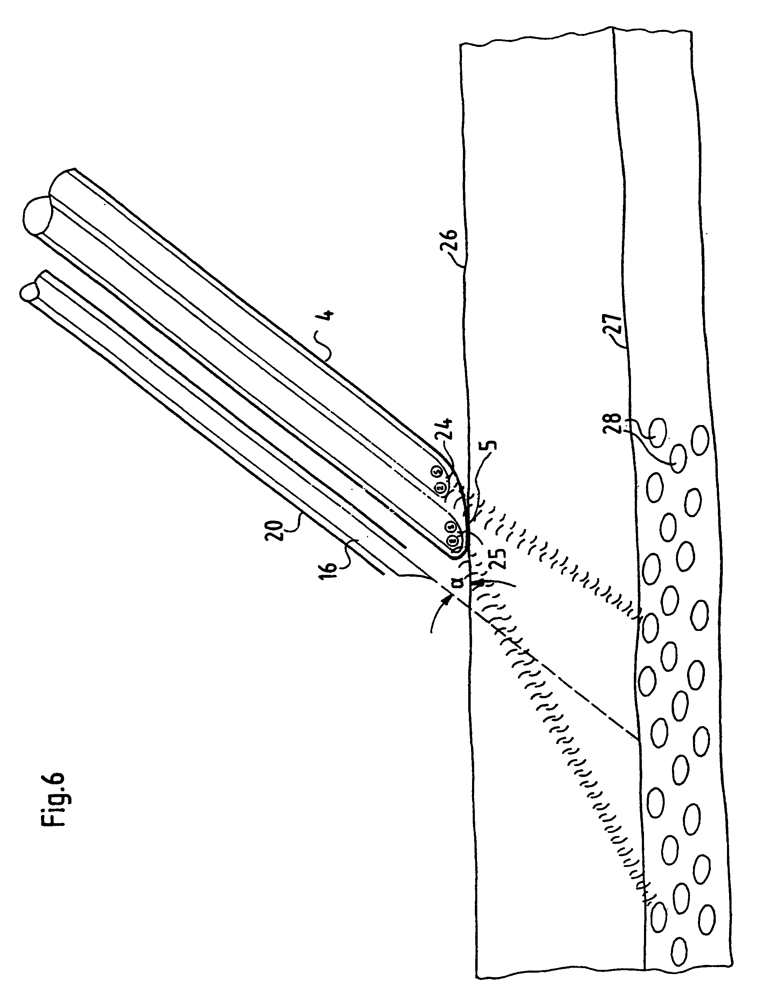

Unmittelbar hinter der Anlagefläche 5 sind innerhalb des Gehäuses 1 zwei Ultraschallortungseinrichtungen 24 und 25 angeordnet, so wie dies in Fig. 6 schematisch dargestellt ist. Die Ortungseinrichtungen bestehen entweder, wie in der dargestellten Ausführung, jeweils aus einem Ultraschallsender S und einem Ultraschallempfänger E oder aus einer Kombination von Sender und Empfänger (Pulsbetrieb). Dabei wird das empfangene Reflexionssignal der Ultraschallwellen nicht nur wie bei der üblichen Ultraschallortung hinsichtlich der zeitlichen Abstände der zurückkommenden Signale ausgewertet, sondern darüber hinaus auch hinsichtlich der Frequenzverschiebung, die sich nach dem Dopplereffekt ergibt, wenn die Ultraschallwellen an bewegten Körpern, insbesondere den Blutkörperchen eines Blutgefäßes reflektiert werden. In Fig. 6 ist dies schematisch dargestellt, dabei ist die Hautoberfläche mit 26, das zu ortende Gefäß mit 27 und die darin strömenden Blutkörperchen mit 28 beziffert. Die Ultraschallortungseinrichtungen 24 und 25 sind so ausgerichtet, dass sie nicht nur die zu erwartende Punktionsstelle der Punktionskanüle 9 mit dem Gefäß 27 erfassen, sondern auch den Bereich unmittelbar vor und hinter dieser Stelle, so dass der Gefäßverlauf darstellbar ist. Die Auswertung der Signale der Ultraschallortungseinrichtungen 24 und 25 erfolgt über eine in den Figuren nicht dargestellte Steuerung, die innerhalb des Gehäuses 1 angeordnet ist. Die Spannungsversorgung des Gerätes erfolgt über ein Netzkabel 29 oder eingebaute Energiespeicher (z. B. Akkus). Innerhalb des pistolenförmigen Gehäuses 1 befinden darüber hinaus die zum Verfahren der Stangen 7 und 11 erforderlichen elektrischen Antriebe mit gegebenenfalls zwischengeschalteten mechanischen Getriebe.

Das distale Ende 4 des laufähnlichen Teils 3 wird durch die Anlagefläche 5 abgeschlossen, die, wie Fig. 6 zeigt, leicht ballig ausgebildet ist, um einerseits eine leichte Ausrichtung des Gehäuses 1 in Bezug auf die Hautoberfläche 26 zu ermöglichen. Andererseits jedoch ist die Anlagefläche 5 so abgeflacht ausgebildet, dass beim Anlegen derselben an die Hautoberfläche 26 ein sicherer Halt in einer Winkelstellung gegeben ist, die der zu erwartenden optimalen Winkelstellung in etwa entspricht. Dieser Winkel α (Winkel zwischen Verfahrrichtung der Kanülen 9, 15 und der Anlagefläche) beträgt im Ausführungsbeispiel 35°, liegt jedoch üblicherweise zwischen 30° und 45°, je nach Anwendung.

Vor Beginn eines Einführvorganges wird das Gerät mit einer Kanülenanordnung gemäß Fig. 3 bestückt, so dass sich die anhand der Figuren 1 und 2 dargestellte und vorbeschriebene Ausgangsstellung ergibt. Das Gerät erfasst mittels eines an seiner Oberseite, dort, wo die Flügel 14 der Verweilkanüle 15 nach Einsetzen in die zugehörige Halterung angeordnet sind, angeordneten (in den Figuren nicht dargestellten) Barcodelesers einen auf den Flügeln 14 befindlichen Barcode. Aufgrund dieser im Barcode verschlüsselten Informationen werden der Steuerung Typ und Größe der eingesetzten Kanüle übermittelt.

Zum Einführen der Kanüle umfasst die Bedienperson den Griffteil 2 des Gehäuses 1 mit der Hand und setzt das distale Ende 4 mit der Auflagefläche 5 dort auf die Hautoberfläche 26, wo darunter ein Gefäßverlauf zu erwarten ist. Aufgrund der Dopplerauswertungen können die Ortungseinrichtungen 24 und 25 Blutgefäße auffinden. Sobald ein Blutgefäß durch eine der beiden Ortungseinrichtungen 24 oder 25 detektiert worden ist, leuchtet eine erste Leuchtdiode 30 an der Oberseite des Gehäuses 1 auf. So lange diese Diode 30 nicht aufleuchtet, muss mit der Anlagefläche 5 auf der Hautoberfläche 26 verfahren werden, bis ein geeignetes Gefäß 27 geortet wird. Wenn die Leuchtdiode 30 aufleuchtet, muss das Gerät 1 lediglich noch in die für das Einführen der Nadel vorgesehene Winkelstellung und Richtung gebracht werden. Dies ist dann der Fall, wenn beide Ortungseinrichtungen 24 und 25 dasselbe Gefäß detektieren und sich das Gerät im bestimmungsgemäßen Winkeln zum Gefäß 27 befindet. Dann leuchtet eine zweite Leuchtdiode 31 auf, gleichzeitig wird eine Auslösetaste 32 freigeschaltet, so dass das programmgesteuerte Einführen der Kanülen ausgelöst wird.

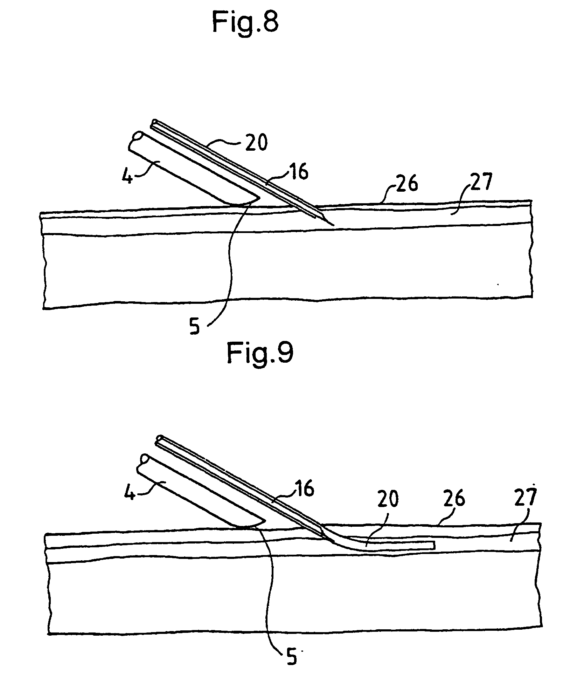

Wenn also die Bedienperson anhand des Signals der Leuchtdioden 30 und 31 erkennt, dass die bestimmungsgemäße Position in Bezug auf das zu punktierende Gefäß 27 erreicht ist, wird die Auslösetaste 32 betätigt. Danach werden zunächst die Stangen 7 und 11 gleichzeitig ausfahren, wodurch die Hautoberfläche durchstochen wird. Die Stangen 7 und 11 werden unter Kontrolle der Ortungseinrichtungen 24 und 25 so lange ausgefahren, bis sowohl das distale Ende der Punktionskanüle 9 als auch das distale Ende der Verweilkanüle 15 das Innere des Gefäßes 27 erreicht haben. Sodann werden die Stangen 11 weiter ausgefahren, die Stangen 7 jedoch gleichzeitig oder anschließend zurückgezogen (siehe Fig. 9). Die Punktionskanüle 9 wird mittels der Stangen so weit zurückgezogen, dass sie die Verweilkanüle 15 proximalseitig gerade noch verschließt.

Das weitere Entfernen der Punktionskanüle 9 erfolgt manuell, da dann die Verweilkanüle proximalseitig ohnehin mit einem Verschlussstopfen versehen oder angeschlossen werden muss. Es versteht sich, dass die Halterungen 8 und 13 ebenfalls zu lösen sind, um das Gerät von den Kanülen zu trennen. Dies kann in einer Weiterbildung ebenfalls Teil der Programmsteuerung sein. In der dargestellten Ausführung sind die Halterungen jedoch als Klemmhalterungen ausgebildet, so dass sie durch leichte Kraftanwendung mit der freien Hand gelöst werden können.

Es versteht sich, dass mit der vorbeschriebenen Vorrichtung nicht nur Verweilkanülen, sondern auch Punktionskanülen, wie sie beispielsweise unmittelbar an Spritzen angeschlossen werden, in ein Gefäß eingebracht werden können. Grundsätzlich genügt eine rechnerische Auswertung der Ausgangssignale der Ortungseinrichtungen 24 und 25, um eine sichere Steuerung der Vorrichtung zu ermöglichen und die bestimmungsgemäße Punktion des Gefäßes zu erreichen. Es können jedoch neben den hier beschriebenen einfachen Signalgebern 30 und 31 sowohl akustische Signalgeber als Ortungshilfen als auch komplexe optische Hilfen, beispielsweise in Form eines Koordinatenkreuzes mit vier Leuchtdioden an den Enden zur leichteren Positionierung vorgesehen sein. Für besonders komplizierte Eingriffe in tiefer gelegene Gefäße kann auch eine zusätzliche Monitorkontrolle vorgesehen sein, wie dies für Ortungseinrichtungen der vorbeschriebenen Art bekannt ist.

### Bezugszeichenliste

- 1 -: pistolenförmiges Gehäuse
- 2 -: Griffteil
- 3 -: laufähnlicher Teil
- 4 -: distales Ende von 1
- 5 -: Anlagefläche
- 6 -: Führung
- 7 -: Stangen
- 8 -: Halterung
- 9 -: Punktionskanüle
- 10 -: Führung
- 11 -: Stangen
- 12 -: Mitnehmer
- 13 -: Halterungen
- 14 -: Flügel
- 15 -: Verweilkanüle
- 16 -: Rohr
- 17 -: Kunststoffteil
- 18 -: Verschlussstopfen
- 19 -: Führungsteil
- 20 -: Kunststoffrohr
- 21 -: Kunststoffteil
- 22 -: Querzugang
- 23 -: Verschlussstopfen
- 24 -: Ultraschallortungseinrichtung
- 25 -: Ultraschallortungseinrichtung
- 26 -: Hautoberfläche
- 27 -: Gefäß
- 28 -: Blutkörperchen
- 29 -: Kabel
- 30 -: erste Leuchtdiode
- 31 -: zweite Leuchtdiode
- 32 -: Auslösetaste

- α -: Winkel zwischen Verfahrrichtung der Stangen 7 und 11 und der Anlagefläche 5

## Patentansprüche

1. Vorrichtung zum Einführen einer elastisch biegsamen Verweilkanüle (16) zusammen mit einer darin befindlichen im Wesentlichen starren Punktionskanüle (9) in ein Blutgefäß (27) eines menschlichen oder tierischen Körper mit folgenden Merkmalen:
- mindestens eine Ortungseinrichtung (24, 25) zum Auffinden des Gefäßes (27) und zur Erfassung des Gefäßverlaufs vor und hinter der Punktionsstelle bzw. der zu erwartenden Runktionsstelle der Punktionskanüle (9, 16),
- mindestens eine Führung (6, 10) für die einzuführenden Kanülen (9, 15),
- Halterungen (8, 13), mit der die Kanülen (9, 15) an der Vorrichtung befestigbar sind, und die in Einführrichtung sowie in Gegenrichtung verschiebbar gelagert sind,
- Antriebsmittel (6, 7; 10, 11) zum Bewegen der Halterungen (8, 13) längs der Führung (6, 10) derart, dass die Verweilkanüle (16) getrennt von der Punktionskanüle (9) verfahrbar ist,
- eine Steuerung, welche die Antriebsmittel zum Einführen der distalen Kanülenenden (16, 20) in das Gefäß (27) in Abhängigkeit des Ausgangssignals der Ortungseinrichtung (24, 25) sowie zum nachfolgende Herausziehen der Punktionskanüle (9) aus der Verweilkanüle (16) ansteuert.

2. Vorrichtung nach Anspruch 1, **dadurch gekennzeichnet, dass** zwei vorzugsweise dopplersonographisch arbeitende Ortungseinrichtungen (24,25) vorgesehen sind, von denen eine den Bereich vor und die andere den Bereich hinter der Punktionsstelle bzw. der zu erwartenden Punktionsstelle erfasst.

3. Vorrichtung nach einem der vorhergehenden Ansprüche, **dadurch gekennzeichnet, dass** die Steuerung den die Kanülen (9, 16) in Einführrichtung bewegenden Antrieb nur dann freigibt, wenn die Auswertung der von den Ortungseinrichtungen (24, 25) abgegebenen Signale durch die Steuerung ergibt, dass sich die Vorrichtung in einer für den Punktionsvorgang geeigneten Stellung zu dem zu punktierenden Gefäß (27) befindet.

4. Vorrichtung nach einem der vorhergehenden Ansprüche, **dadurch gekennzeichnet, dass** eine optische und/oder akustische Anzeige (30, 31) vorgesehen ist, welche mindestens dann ein Signal abgibt, wenn sich die Vorrichtung in der bestimmungsgemäßen Lage zur Punktion des georteten Gefäßes (27) befindet.

5. Vorrichtung nach einem der vorhergehenden Ansprüche, **dadurch gekennzeichnet, dass** das distale Ende (4) der Vorrichtung eine vorzugsweise ballig ausgebildete Anlagefläche (5) aufweist, die in Bezug auf die Bewegungsrichtung der Kanülenhalterung (8, 13) schräggestellt ist, und zwar vorzugsweise in einem Winkel zwischen 30° und 45°.

6. Vorrichtung nach einem der vorhergehenden Ansprüche, **dadurch gekennzeichnet, dass** eine Linearführung (6) für die Punktionskanüle (9) und eine weitere Linearführung (10) parallel dazu für die Verweilkanüle (16) vorgesehen sind.

7. Vorrichtung nach einem der vorhergehenden Ansprüche, **dadurch gekennzeichnet, dass** die Vorrichtung im wesentlichen in Form einer Pistole (1) ausgebildet ist und eine Auslösetaste (32) im vorderen Teil des Griffstücks (2) aufweist, wobei die Steuer- und Auswertelektronik vorzugsweise innerhalb des Griffstücks (2) angeordnet sind.

8. Vorrichtung nach einem der vorhergehenden Ansprüche, **dadurch gekennzeichnet, dass** jeder Führung (6, 10) ein elektrischer Antrieb in Form eines Elektromotors zugeordnet ist, die vorzugsweise innerhalb des Griffstücks (2) angeordnet sind.

9. Vorrichtung nach einem der vorhergehenden Ansprüche, **dadurch gekennzeichnet, dass** Mittel zur Erfassung von Kanülentyp und/oder-größe vorgesehen sind.

10. Vorrichtung noch einem der vorhergehenden Ansprüche, **dadurch gekennzeichnet, dass** die Elektromotoren von der Steuerung so angesteuert werden, dass zunächst die Verweilkanüle (16) zusammen mit der Punktionskanüle (9) distalwärts verfahren wird, dass nach Punktion des Gefäßes (27) und nach Durchdringen der Gefäßwand durch die Verweilkanüle (16) diese weiter distalwärts verfahren wird und die Punktionskanüle (9) gleichzeitig oder anschließend zurückgezogen wird.

## Claims

1. A device for introducing an elastically flexible in-dwelling cannula (16) together with an essentially rigid puncture cannula (9) located therein into a blood vessel (27) of a human or animal body, with the following features:
- at least one locating means (24, 25) for locating the vessel (27) and for detecting the course of the vessel in front of and behind the puncture location or the expected puncture location of the puncture cannula (9, 16),
- at least one guide (6, 10) for the cannulas (9, 15) which are to be introduced,
- mountings (8, 13) with which the cannulas (9, 15) may be fastened on the device and which are displaceably guided in the introduction direction as well as in the opposite direction,
- drive means (6,7;10,11) for moving the mountings (8, 13) along the guide (6, 10) in a manner such that the in-dwelling catheter (16) may be travelled separately from the puncture cannula (9),
- a control, which activates the drive means for introducing the distal cannula ends (16, 20) into the vessel (27) in dependence on the output signal of the locating means (24, 25) as well as for the subsequent retraction of the puncture cannula (9) from the in-dwelling cannula (16).

2. A device according to claim 1, **characterised in that** two, preferably doppler-sonographically functioning locating means (24, 25) are provided, of which one detects the region in front and the other the region behind the puncture location or the expected puncture location.

3. A device according to one of the preceding claims, **characterised in that** the control only releases the drive which moves the cannulas (9, 16) in the introduction direction when the evaluation of the signals emitted by the locating means (24, 25) by way of the control results that the device is located in a position to the vessel (27) to be punctured which is suitable for the puncture procedure.

4. A device according to one of the preceding claims, **characterised in that** an optical and/or acoustic display (30, 31) is provided which emits a signal at least when the device is located in the correct position for puncture of the located vessel (27).

5. A device according to one of the preceding claims, **characterised in that** the distal end (4) of the device comprises a bearing surface (5) formed preferably in a convex manner, which is set obliquely with respect to the movement direction of the cannula mounting (8, 13), and specifically at an angle between 30° and 45°.

6. A device according to one of the preceding claims, **characterised in that** a linear guide (6) for the puncture cannula (9) and a further linear guide (10) parallel thereto for the in-dwelling catheter (16) are provided.

7. A device according to one of the preceding claims, **characterised in that** the device is formed essentially in the shape of a pistol (1) and comprises a trigger button (32) in the front part of the grip piece (2), wherein the control and evaluation electronics are preferably arranged within the grip piece (2).

8. A device according to one of the preceding claims, **characterised in that** an electrical drive in the form of an electric motor which is preferably arranged within the grip piece (2) is allocated to each guide (6, 10).

9. A device according to one of the preceding claims, **characterised in that** means for detecting the cannula type and/or cannula size are provided.

10. A device according to one of the preceding claims, **characterised in that** the electric motors are activated by the control such that firstly the in-dwelling cannula (16) together with the puncture cannula (9) are travelled distally, that after puncture of the vessel (27) and after the penetration of the vessel wall by the in-dwelling cannula (16) this is travelled further distally and the puncture cannula (9) is simultaneously or subsequently retracted.

## Revendications

1. Dispositif pour l'introduction d'une canule de séjour (16) élastiquement flexible conjointement avec une canule de ponction (9) sensiblement rigide et se trouvant à l'intérieur dans un vaisseau sanguin (27) d'un corps humain ou animal présentant les caractéristiques suivantes :
- au moins un dispositif de localisation (24, 25) pour trouver le vaisseau (27) et pour enregistrer le tracé du vaisseau devant et derrière le point de ponction ou le point de ponction à escompter de la canule de ponction (9, 16),
- au moins un guide (6, 10) pour les canules (9, 15) à introduire,
- des attaches (8, 13), avec lesquelles les canules (9, 15) peuvent être fixées sur le dispositif, et qui sont logées de façon coulissante dans le sens d'introduction et dans le sens contraire,
- des moyens d'entraînement (6, 7 ; 10, 11) pour le déplacement des attaches (8, 13) le long du guide (6, 10) de telle sorte que la canule de séjour (16) peut être déplacée séparément de la canule de ponction (9),
- une commande qui active des moyens d'entraînement pour l'introduction des extrémités de canule (16, 20) distales dans le vaisseau (27) en fonction du signal de sortie du dispositif de localisation (24, 25) et pour l'extraction consécutive de la canule de ponction (9) de la canule de séjour (16).

2. Dispositif selon la revendication 1, **caractérisé en ce que** deux dispositifs de localisation (24, 25) travaillant de préférence avec la sonographie de Doppler sont prévus, dont l'un enregistre la zone située avant le point de ponction et l'autre la zone située derrière ou le point de ponction à escompter.

3. Dispositif selon l'une quelconque des revendications précédentes, **caractérisé en ce que** la commande ne libère l'entraînement déplaçant les canules (9, 16) dans le sens d'introduction que dans le cas où l'analyse par la commande des signaux émis par les dispositifs de localisation (24, 25) fait que le dispositif se trouve dans une position appropriée pour l'opération de ponction par rapport au vaisseau (27) faisant l'objet d'une ponction.

4. Dispositif selon l'une quelconque des revendications précédentes, **caractérisé en ce qu'**un affichage (30, 31) optique et/ou acoustique est prévu, qui émet un signal au moins dans le cas où le dispositif se trouve dans la position conforme à l'affectation pour la ponction du vaisseau (27) détecté.

5. Dispositif selon l'une quelconque des revendications précédentes, **caractérisé en ce que** l'extrémité (4) distale du dispositif présente une surface d'appui (5) conçue de préférence bombée, qui est inclinée par rapport au sens de déplacement du support de canule (8, 13), et ce de préférence dans un angle compris entre 30° et 45°.

6. Dispositif selon l'une quelconque des revendications précédentes, **caractérisé en ce qu'**un guide linéaire (6) est prévu pour la canule de ponction (9) et un autre guide linéaire (10) est prévu parallèlement au premier pour la canule de séjour (16).

7. Dispositif selon l'une quelconque des revendications précédentes, **caractérisé en ce que** le dispositif est conçu sensiblement sous la forme d'un pistolet (1) et présente une touche de déclenchement (32) dans la partie avant de la poignée, l'électronique de commande et l'électronique d'analyse étant disposées de préférence à l'intérieur de la poignée (2).

8. Dispositif selon l'une quelconque des revendications précédentes, **caractérisé en ce qu'**un entraînement électrique sous la forme d'un moteur électrique, qui est disposé de préférence à l'intérieur de la poignée (2), est attribué à chaque guide (6, 10).

9. Dispositif selon l'une quelconque des revendications précédentes, **caractérisé en ce que** des moyens sont prévus pour l'enregistrement du type de canule et/ou de la grandeur de canule.

10. Dispositif selon l'une quelconque des revendications précédentes, **caractérisé en ce que** les moteurs électriques sont actionnés par la commande de telle sorte que d'abord la canule de séjour (16) est déplacée conjointement avec la canule de ponction (9) vers le côté distal, **en ce que**, après la ponction du vaisseau (27) et après la pénétration de la paroi du vaisseau à travers par la canule de séjour (16), celle-ci est déplacée encore vers le côté distal et la canule de ponction (9) est reculée simultanément ou ensuite.
